# EUROPEAN PATENT APPLICATION

(11) **EP 4 470 549 A1**
(43) Date of publication of application: **04.12.2024**
(21) Application number: 22923687.2
(22) Date of filing: 19.10.2022
(51) Int. Cl.: A61K 36/14, A61K 36/61, A61K 8/9761, A61K 8/9789, A61P 1/02, A61P 31/02, A61Q 11/00

(54) **NATURAL ANTISEPTIC SOLUTION FOR ORAL HYGIENE AND METHOD FOR PRODUCING SAME**

(30) Priority: 31.01.2022 ES 202230073
(71) Applicant: Herrera Val, Zoraida Isabel, 28053 Madrid (ES)
(72) Inventor: Herrera Val, Zoraida Isabel, 28053 Madrid (ES)
(74) Representative: Díaz de Bustamante y Terminel, Isidro
(86) International application number: PCT/ES2022/070667
(87) International publication number: WO 2023/144426

(57) **Abstract**

NATURAL ANTISEPTIC SOLUTION FOR ORAL HYGIENE AND PROCEDURE FOR OBTAINING THEREOF which consists of a product whose composition is a mixture of natural substances, based on edible plants, and an eluent that is edible alcohol, in addition to water; being the following in the following proportions: 0.3% Juniper (Juniperus Communis), 0.4% Clove (Syzygium aromaticum), 0.4% Punica granatum Bark, 50% Ethyl Alcohol and 48.9% Sterile Water, and can be presented, for application, in the form of a nebulizer, a bottle for gargling or a dropper for instilling into the nostrils. For its obtention, it comprises, at least: a step of mixing all the plant components with alcohol and water, and a resting step for between four and five weeks away from light at a temperature not exceeding 21°.

## Description

### OBJECT OF THE INVENTION

The invention, as exposed on the wording of the present specification, refers to a natural antiseptic solution for oral hygiene and to the procedure for obtaining thereof, which impart to the function it is intended to, advantages and characteristics, which are described in detail below, which are an improvement of the current state of the art.

More specifically, the object of the invention focuses, on the one hand, on a liquid solution which, intended for use as a mouthwash for rinsing the mouth, is distinguished by the fact that it consists of a product whose composition is, advantageously, a mixture of substances in which absolutely all of them are natural, specifically based on edible plants of proven harmlessness and non-toxicity, constituting an effective complement to sanitize the mouth, nasopharynx and dental interstices to prevent the development of fungi, viruses and bacteria in a comfortable and absolutely harmless way. A second aspect of the invention relates to the procedure for obtaining said antiseptic solution.

### APPLICATION FIELD OF THE INVENTION

The field of application of the present invention is framed within the sector of the industry dedicated to the manufacture of oral hygiene products.

### BACKGROUND OF THE INVENTION

There are known on the market many solutions that can be used, such as the one here concerned, for oral hygiene, with the aim of, among other things, trying to prevent the development of fungi, viruses and bacteria that can lead to health problems. The problem is that most of the solutions and mouthwashes currently known, in order to ensure adequate effectiveness, include, in their compositions, products and/or chemical substances, antibiotics and medicines that are not always desirable for the consumer.

On the other hand, there are also all kinds of solutions for mouthwashes made from natural products. In this case, the problem is usually that they are not an effective product to correctly or completely prevent the eventual development of fungi, viruses and bacteria that affect the different parts of the mouth, pharynx, and dental structure.

The objective of the present invention is, therefore, to provide a new product that provides the effectiveness of products specifically intended to prevent the formation of any type of germs and, at the same time, is totally natural and harmless so that it can be used without problems by any user.

Furthermore, and as a reference to the current state of the art, it should be noted that, although as has been indicated there are other substances with similar applications, at least the applicant is unaware of the existence of any that have technical characteristics and composition equal or similar to those presented by the one claimed here.

### EXPLANATION OF THE INVENTION

The natural antiseptic solution for oral hygiene proposed by the invention represents an improvement over what is already known, and the characterising details that make it possible and that distinguish it are conveniently included in the final claims that accompany the present description.

What the invention proposes, as has been pointed out above, is a liquid solution intended for use as a mouthwash for rinsing the mouth, which is distinguished by the fact that it consists of a product whose composition is, advantageously, a mixture of natural substances, specifically based on edible plants, constituting an effective complement to sanitise the mouth, nasopharynx and dental interstices to prevent the development of fungi, viruses and bacteria in a comfortable and absolutely harmless way.

More specifically, to obtain the solution object of the invention, only edible plants are used, of proven harmlessness and non-toxicity, and elution as a procedure for mixing in an eluent that is edible alcohol.

Preferably, the solution, in addition to sterile water and ethyl alcohol, comprises the following substances as natural substances:
- Juniper (Juniperus communis).
- Clove (Syzygium aromaticum).
- Punica granatum husk.

And, in a preferred embodiment, the solution object of the invention comprises the aforementioned substances incorporated in the following proportions:
- 0.3% Juniper (Juniperus communis).
- 0.4% Clove (Syzygium aromaticum).
- 0.4% Punica granatum husk.
- 50% Ethyl alcohol.
- 48.9% Sterile water.

As regards the procedure for obtaining the antiseptic solution, this comprises at least the following steps:
- A step of mixing all the plant components with the alcohol and water, in the aforementioned proportions.
- And a resting step of said mixture for a period of between four and five weeks away from light and at a temperature not exceeding 21°.

It should be noted that laboratory and field tests have been carried out over a period of twenty months, giving optimal results due to its great capacity to sanitize, and inhibit viruses, bacteria and fungi (not spores) capable of causing infections in the oral, periodontal and nasopharynx mucosa, as well as preventing them from proliferating right there at the entrance and from being able to pass to the lower respiratory tract.

Thus, the application of the solution object of the invention is, basically, for use as an effective complement to sanitize the mouth, nasopharynx, and dental interstices, preventing the development of fungi, viruses, and bacteria, in a comfortable and absolutely harmless way.

Finally, it should be mentioned that, preferably, the solution is presented in at least three different format options for use or application:
- In a nebulizer, so that it can be easily carried around (bag, jacket, etc.)
- In a bottle for gargling after daily oral hygiene, or after a meeting or being in a closed office with many people.
- In the form of a dropper to be instilled into the nostrils.

Having sufficiently described the nature of the present invention, as well as a way of putting it into practice, it is not considered necessary to make a more extensive explanation in order that any person skilled in the art will understand its scope and the advantages derived from it, making known that, within reason it could be put into practice in other embodiments differing in detail from that indicated by way of example, and which will obtain the degree of protection that is sought, provided that its fundamental principle is not altered, changed or modified.

## Claims

1. NATURAL ANTISEPTIC SOLUTION FOR ORAL HYGIENE which is a composition **characterized by** consisting of a mixture of edible alcohol that acts as an eluent, in addition to water and the following natural substances (based on edible plants):
- Juniper (Juniperus communis),
- Clove (Syzygium aromaticum),
- Punica granatum husk.

2. NATURAL ANTISEPTIC SOLUTION FOR ORAL HYGIENE, according to claim 1, **characterized in that** it comprises the aforementioned substances incorporated in the following proportions:
- 0.3% Juniper (Juniperus communis),
- 0.4% Clove (Syzygium aromaticum),
- 0.4% Punica granatum husk,
- 50% Ethyl alcohol,
- 48.9% Sterile water.

3. NATURAL ANTISEPTIC SOLUTION FOR ORAL HYGIENE, according to claim 1 or 2, **characterized in that**, for its application, it is presented in the form of a nebulizer.

4. NATURAL ANTISEPTIC SOLUTION FOR ORAL HYGIENE, according to claim 1 or 2, **characterized in that**, for its application, it is presented in the form of a bottle for gargling.

5. NATURAL ANTISEPTIC SOLUTION FOR ORAL HYGIENE, according to claim 1 or 2, **characterized in that**, for its application, it is presented in the form of a dropper to be instilled into the nostrils.

6. PROCEDURE FOR OBTAINING an antiseptic solution as described in claims 1 or 2, **characterized by** comprising at least:
- a step of mixing all the plant components with alcohol and water,
- and a step of allowing said mixture to rest for a period of between four and five weeks away from light and at a temperature not exceeding 21°.
